# EUROPÄISCHE PATENTSCHRIFT

(11) **EP 1 391 205 B1**
(45) Veröffentlichungstag und Bekanntmachung des Hinweises auf die Patenterteilung: **28.10.2009**
(21) Anmeldenummer: 03016127.7
(22) Anmeldetag: 16.07.2003
(51) Int. Cl.: A61K 31/7088, A61K 31/7105, A61K 31/713, A61K 38/46, A61P 7/02, A61P 7/04

(54) **Pharmazeutische Zubereitung mit RNA als Cofaktor der Hämostase**
Pharmaceutical composition with RNA as cofactor of hemostasis
Composition pharmaceutique comprenant de l'ARN en tant que cofacteur de l'hémostase

(30) Priorität: 06.08.2002 DE 10236038; 03.03.2003 DE 10309368
(43) Veröffentlichungstag der Anmeldung: 25.02.2004
(73) Patentinhaber: CSL Behring GmbH, 35041 Marburg (DE)
(72) Erfinder: Preissner, Klaus, 35394 Giessen (DE); Nakazawa, Fumie, Tokyo (JP); Kannemeier, Christian, San Diego, CA 92109 (US); Roemisch, Juergen, 2331 Voesendorf (AT)

(56) Entgegenhaltungen:
- EP-A- 0 288 306
- EP-A- 0 670 165
- WO-A-01/00829
- WO-A-01/08634
- WO-A-01/73003
- WO-A-98/51335
- WO-A-02/096926
- DE-A- 19 903 693
- US-A- 5 141 849
- DATABASE WPI Section Ch, Week 198652 Derwent Publications Ltd., London, GB; Class B04, AN 1986-345695 XP002255128 & SU 1 232 263 A (DOMESS MED PIROGOV), 23. Mai 1986 (1986-05-23)

## Beschreibung

Gegenstand der Erfindung sind pharmazeutische Zubereitungen und Verwendungen dieser Zubereitungen, die neben Blutgerinnungsfaktoren zusätzlich noch natürliche oder synthetische Ribonukleinsäure (RNA) oder biologisch aktive Bruchstücke der RNA oder RNA-degradierende Substanzen wie Ribonukleasen enthalten.

Es ist bekannt, dass die Prozesse der Hämostase (Blutgerinnung und Fibrinolyse), die lokal und zeitlich begrenzt nach Einsetzen einer Gefäßläsion ablaufen, durch spezifische Interaktionen zwischen aktivierten Gefäßwandzellen, Blutplättchen und Proteinfaktoren gesteuert werden. Über zumeist ionische Wechselwirkungen der Calzium-bindenden und Vitamin K-abhängigen Gerinnungsfaktoren wird eine Lokalisierung der in der Hämostase wirksamen Proteine auf vor allem negativ geladenen Biomembranen an der Verletzungsstelle erreicht. In vitro können diese Wechselwirkungen und damit die Aktivierung des Blutgerinnungssystems sowie das Ausmaß der Bildung eines Fibringerinnsels durch komplexierende Substanzen wie Ethylendiamin-tetraessigsäure (EDTA) oder Citrat verhindert werden. In vivo verbietet sich der Einsatz solcher Antikoagulantien; hier kann über die Therapie mit oralen Vitamin K-Antagonisten die Calziumbindung der Gerinnungsfaktoren verhindert werden, so dass das Ausmaß der Hämostase herabgesetzt wird.

Neben der Bildung von negativ geladenen Phospholipiden durch Freilegung von Zellmembranen der Blutplättchen und anderer vaskulärer Zellen kommt es nach Verletzung der Gefäßwand auch zur Exposition intrazellulärer Komponenten, insbesondere der zytosolischen Proteine. Gut erforscht ist, dass das Blutgerinnungssystem zwei unterschiedliche, kaskadenförmige Aktivierungswege von im Plasma anwesenden Gerinnungsfaktoren umfasst. Je nach auslösendem Mechanismus dient bevorzugt der endogene oder der exogene Weg zur Initiation der Gerinnung. Bei einer Gewebeverletzung wird als Starter des exogenen Gerinnungsweges das Thromboplastin (tissue factor, TF) mit Phospholipiden von den betroffenen Zellen exponiert. Die besondere Funktion des Blutgerinnungsfaktors VII bei diesen Vorgängen ist aus der deutschen Offenlegungsschrift 199 03 693 bekannt. Das membranständige Thromboplastin kann sowohl den Gerinnungsfaktor VII (FVII) als auch den zirkulierenden, aktivierten FVII (FVIIa) binden. Dieser TF-FVIIa-Komplex führt in Gegenwart von Calzium-lonen und Lipiden zur Bindung des FX, der durch limitierte Proteolyse in seine aktivierte Form (FXa) überführt wird. FXa wiederum führt durch Aktivierung von Prothrombin zu Thrombin zur Bildung von Fibrin und damit letztendlich zum Wundverschluss.

Nach den bisherigen Kenntnissen vollzieht sich die weitere Aktivierung des an das Thromboplastin gebundenen FVII vor allem autokatalytisch, wird aber nach der Initiation der Gerinnungskaskade vor allem durch FXa und Thrombin unterstützt, was zu einer deutlichen Verstärkung der Reaktionskaskade führt.

Aus diesen Befunden konnte die Erkenntnis abgeleitet werden, dass in bestimmten klinischen Situationen die Applikation von FVIIa oder FVIIa-enthaltenden Konzentraten indiziert ist. Bei Patienten, die zum Beispiel unter Hämophilie A leiden und als Folge der Verabreichung von FVIII Antikörper gegen FVIII entwickelt haben, wird die sog. "FVIII inhibitor bypassing activity" (FEIBA) des FVIIa genutzt. Dabei hat sich gezeigt, dass FVIIa gut verträglich ist und zu keiner Thromboseneigung führt, sich aber dazu eignet, die Gerinnung in einem begrenzten, jedoch ausreichendem Umfang zu gewährleisten. Rekombinanter FVIIa wird bereits therapeutisch und prophylaktisch verwendet. Aus Blutplasma gewonnener FVII kann ebenfalls aktiviert und danach verwendet werden. Zu dieser Aktivierung können nach den bisherigen Kenntnissen Proteasen wie Thrombin verwendet werden, die aber als solche die Gerinnung selbst stark aktivieren und zu einer Thrombosegefährdung führen können. Deshalb ist die anschließende Entfernung oder Inaktivierung von Thrombin erforderlich und führt zu Ausbeuteverlusten. Die Anwendung von FXa oder Flla (Thrombin) ist wegen der damit verbundenen Thrombosegefährdung häufig kontraindiziert und nur in Notfällen, zum Beispiel bei extremen Blutverlusten und unstillbaren Blutungen angezeigt.

FVIIa wird nur in sehr geringen Konzentrationen im Plasma gesunder Menschen gefunden. Über die Bildung und Herkunft des im Blut zirkulierendenFVIIa ist bisher nur sehr wenig bekannt. Es wurde deshalb angenommen, dass Spuren von exprimiertem oder bei einer Zellzerstörung freigesetztem Thromboplastin dabei eine Rolle spielen könnten. Trotz der intensiven Erforschung aller mit der Blutgerinnung zusammenhängenden Vorgänge konnten bisher jedoch keinerlei Hinweise dafür gefunden werden, dass Bestandteile der verletzten Zelle bei der Auslösung der hämostatischen Prozesse eine entscheidende Rolle spielen könnten.

Überraschenderweise wurde nun gefunden, dass unter den aus verletzten Geweben und Zellen freigesetzten Bestandteilen die extrazelluläre RNA einen wichtigen initialen Cofaktor für das Auslösen der (a) extrinsischen und (b) intrinsischen Gerinnungskaskade darstellt. Diese Beobachtung gibt Anlass, die Funktion der RNA oder von biologisch aktiven Bruchstücken der RNA bzw. von RNAsen in hämostatischen Prozessen genauer zu untersuchen und pharmazeutische Zubereitungen zu entwickeln, denen zur Förderung der Hämostase natürliche oder synthetische RNA oder biologisch aktive Bruchstücke der RNA, oder denen zur Inhibierung von extrazellulären Funktionen von RNA entsprechende Ribonukleasen zugesetzt sind. Extrazelluläre RNA stellt dabei die natürliche "fremde" Oberfläche zur Aktivierung des plasmatischen Kontaktphase Systems dar, dessen initiierung bislang durch Kaolin oder andere polyanionische Substanzen in vitro beschrieben wurde.

Gegenstand der Erfindung ist deshalb eine pharmazeutische Zubereitung, die eine zur Förderung der Blutgerinnung ausreichende Menge von natürlicher oder synthetischer RNA oder von einem oder mehreren die Blutgerinnung fördernden Bruchstücken von RNA, Peptidnukleinsäuren, Ribozymen oder RNA-Aptameren enthält. Eine derartige Zubereitung umfasst zusätzlich noch einen Aktivator für einen plasmatischen Blutgerinnungsfaktor. Besonders geeignet als Aktivator ist (a) die den Faktor VII aktivierende Protease (FSAP) oder ihr Proenzym sowie (b) Komponenten der Kontaktphase wie Faktor XII, Kininogen und Präkallikrein. Weiterhin ist Gegenstand der Erfindung die Verwendung einer pharmazeutischen Zubereitung zur Herstellung eines Medikaments enthaltend Ribonukleasen, die die prokoagulatorische Wirkung von RNA aufheben oder verhindern.

Den erfindungsgemäßen pharmazeutischen Zubereitungen liegt die Erkenntnis zugrunde, dass die RNA den wirksamsten Cofaktor für das Proenzym des FSAP darstellt und zur Aktivierung dieses Enzyms führt. Diese Wechselwirkung wird sowohl für natürliche RNA aus Zellhomogenaten oder Überständen aktivierter Thrombozyten als auch mit aus zytosolischer RNA (vor allem ribosomale RNA) gewonnenen Fraktionen oder synthetischer RNA erzielt. Dabei besteht offensichtlich kein zellspezifischer Cofaktoreffekt, da auch bakterielle und virale RNA wirksam ist und sich die RNA-Moleküle in den verschiedenen Zelltypen sehr ähnlich sind, so dass offenbar die hohe negative Überschussladung die Funktionen von RNA als Cofaktor der FSAP bestimmt.

Diese Erkenntnisse werden durch die Beobachtung untermauert, dass FSAP spezifisch an RNA binden kann und durch eine hypertone Kochsalzlösung auch wieder dissoziiert wird. Bemerkenswert ist also, dass unter physiologischen Bedingungen eine spezifische Bindung an FSAP nur durch RNA, nicht aber durch DNA feststellbar ist.

Der Erfindung liegt somit die Erkenntnis zugrunde, dass durch die Wechselwirkung von RNA mit FSAP, welches der wirksamste Aktivator des Gerinnungs-Proenzymsfaktors VII ist, die extrinsische Aktivierung der Blutgerinnung induziert wird. Dieser durch Gewebsverletzungen spezifisch aktivierte Gerinnungsweg wird also durch den neuen Cofaktor (natürliche oder synthetische) RNA in Gang gesetzt.

Gleichzeitig stellt RNA den natürlichen Cofaktor zur Aktivierung des Kontaktphase Systems dar, was im Vollblut, Plasma und im gereinigten System gezeigt wurde. Damit eröffnen sich neue und ganz unterschiedliche Wege durch geeignete pharmazeutische Zubereitungen positiv wie negativ Einfluss auf die Vorgänge der Hämostase zu nehmen.

Ein neuer Ansatzpunkt hierfür ist die Verwendung von RNA-abbauenden und inhibierenden Verbindungen zur Herstellung eines Medikaments zur Einwirkung auf hämostatische Prozesse. Wird durch Zuführung von RNA-spaltenden oder inhibierenden Substanzen der die Blutgerinnung beeinflussende Cofaktor RNA inaktiviert, steht er dann nicht mehr für die Aktivierung von FSAP oder des Kontakphase Systems zur Verfügung. Hieraus ergibt sich, dass RNAsen die Wirkung von FSAP aufheben und damit auch den Blutgerinnungsfaktor VII bzw. die intrinsische Gerinnung unwirksam machen können. Das hat ein neues anticoagulatorisches Prinzip zur Folge, das therapeutisch nutzbar ist. RNA-degradierende oder maskierende Komponenten (wie z.B. Bindungsproteine) können also wichtige therapeutische Wirkungen entfalten, die hochspezifisch, ohne Nebenwirkungen, selektiv die Initiierung des Gerinnungssystems verhindern und damit einen deutlichen anticoagulatorischen oder antithrombotischen Effekt haben.

Die erfindungsgemäßen pharmazeutischen Zubereitungen können als Blutgerinnungsmittel entweder allein oder zusammen mit anderen die Proteaseaktivität erhöhenden Substanzen wie Heparin oder dem Heparin verwandten Substanzen wie Heparansulfat und/oder Calziumionen eingesetzt werden. Die Anwendung eines derartigen Mittels kann zum Beispiel unter Ausnutzung seiner FVIII inhibitor bypassing activity (FEIBA) angezeigt sein, wenn Unverträglichkeiten gegenüber den FVIII und/oder FIX und/oder FXI und/oder den Proteinen der Kontaktphase, wie FXII, zum Beispiel wegen des Vorliegens von Antikörpern, bestehen oder andersartige Mangelsituationen vorliegen. Auch die Anwendung ex vivo zur allgemeinen Blutungsprophylaxe oder zur Stillung von Blutungen kann mit der erfindungsgemäßen pharmazeutischen Zubereitung zur Förderung der Blutgerinnung durchgeführt werden.

Weitere Details der Erfindung werden in folgenden Beispielen verdeutlicht, ohne dass der Gegenstand der Erfindung dadurch in irgendeiner Weise eingeschränkt werden soll:

### Beispiel 1. RNA aus verschiedenen Quellen wirkt als prokoagulatorischer Kofaktor

RNA aus
(a) kultivierten humanen Fibroblasten (rRNA),
(b) Hefe (tRNA),
(c) E. coli,
(d) Qβ-Phagen (Einzelstrang RNA)
(e) synthetischer Quelle (poly IC, poly C, poly AU)
   wurde in gleicher Konzentration ohne und nach Vorbehandlung mit RNAse A in einem turbidometrischen Gerinnungstest eingesetzt und die Rekalzifizierungszeit gemessen. Alle RNA-Typen waren in der Lage, die Gerinnungszeit signifikant zu verkürzen (Quantifizierung in "Kaolin-Equivalent Units (KEU/ml)"), während DNA keine Wirkung zeigte. Vorbehandlung der RNA-Proben mit RNAse A führte zur Inaktivierung der prokoagulatorischen Aktivität; lediglich im Falle von E. coli RNA war diese partiell resistent gegen RNAse A-Behandlung. Damit ist eindeutig gezeigt, dass verschiedene Formen von polyanionischer RNA prokoagulatorische Aktivität besitzen; Fragmentierung dieser RNA-Typen in niedermolekulare Bruchstücke führt zum Verlust der prokoagulatorischen Aktivität. Im Vergleich zu authentischer rRNA oder tRNA zeigten synthetische Einzelstrang- und Doppelstrang-RNA prokoagulatorische Aktivität in Gerinnungstests in der folgender Abstufung: poly IC > poly C > poly AU > poly dldC.

### Beispiel 2. Verhalten von RNA im Plasmamilieu: RNA-Gedächtnis

Obwohl Ribonukleasen im Blut (und in anderen Körperflüssigkeiten) die Menge an zirkulierender RNA kontrollieren, war es möglich, die prokoagulatorische Funktion von RNA im Plasma zu bestimmen. Um diesen Zusammenhang zu klären, wurde das Schicksal einer bestimmten Menge RNA im Plasma über die Zeit verfolgt: Während hochmolekulare RNA im Plasma mit einer Halbwertzeit von ca. 5 min degradiert wurde und nach 30 min nicht mehr nachweisbar war, konnte die prokoagulatorische Aktivität dieser RNA noch nach 90 min und länger unverändert nachgewiesen werden. Dieses Phänomen des "RNA-Gedächtnisses" beruht wahrscheinlich auf der Aktivierung RNA-abhängiger Komponenten im Plasma, die nach Rekalzifizierung mit zur Initiierung der Blutgerinnung beitragen. Sehr wahrscheinlich ist damit RNA im Blutplasma auch verantwortlich für die Phänomene der "latenten" Gerinnung bzw. eines "hyperkoagulabilen" Zustandes, dessen Voraussage bzw. Prävention einen sehr wichtigen Stellenwert in der Diagnose und Therapie von thrombotischen Komplikationen einnimmt.

### Beispiel 3. Mechanismus der RNA-abhängigen Initiierung der Blutgerinnung

Während zum Plasma zugesetzte RNA die durch Tissue factor induzierte Blutgerinnung bei jeder Konzentration dieses Kofaktors steigern konnte, zeigte sich bei Kaolin-induzierter Blutgerinnung ein äquivalenter Effekt der RNA. Dies deutet auf die Beteiligung von RNA als anionischer Kofaktor,(fremde Oberfläche) bei der Initiierung des Kontaktphasesystems der Blutgerinnung hin. Um diesen Sachverhalt zu klären, wurden in einem gereinigten System Präkallikrein und Kininogen zur Reaktion gebracht und die Menge an gebildetem Kallikrein bestimmt. Im Verhältnis zur Pufferkontrolle führte der Zusatz von RNA zu einer signifikanten Steigerung der Kallikreinbildung, während Vorbehandlung der RNA mit RNAse A diesen Effekt verhinderte. Experimente in Faktor XII- und Faktor VIII-defizienten Plasmen unterstrichen die Funktion der RNA als Kontaktphase-Kofaktor, und in Präkallikreindefizientem Plasma zeigte RNA (im Vergleich zu Normalplasma) keine Wirkung. Zusammengenommen beweisen diese Resultate, dass, äquivalent zu Kaolin (artifizielles Material), das natürliche Material RNA nach Exponierung mit dem Kontaktphasesystem die intrinsische Initiierung der Blutgerinnung in Gang setzt.

### Beispiel 4. Nachweis von RNA in Patientenplasma (nicht im Rahmen der Erfindung)

Die Abnahme von Blut erfolgte in der Gegenwart von ca. 200 units/ml RNase Inhibitor mit nachfolgender Präparation des Plasmas oder alternativ wurde nach Blutabnahme und Plasmagewinnung diesem der Stabilisator PAQ-gene (Qiagen) zugesetzt, um RNA-Hydrolyse zu verhindern. Nach Präparation der RNA (mittels Trizolmethode) wurde diese auf Nylonfilter immobilisiert, und mit radioaktiv markierten rRNA-spezifischen Oligos wurde nach Hybridisierung die Menge an Plasma RNA mit Hilfe einer Eichkurve quantifiziert. In Plasmen von Patienten mit akutem Myokardinfarkt oder Sepsis wurden mit Hilfe dieser Analytik positive Proben identifiziert, deren Gehalt an RNA über dem gesunder Spender lag.

### Beispiel 5. RNA-Antagonisten

Die prokoagulatorische Aktivität von RNA, vor allem bezogen auf die Initiierung des plasmatischen Kontaktphasesystems, kann durch Vorbehandlung der RNA mit RNAse A verhindert werden. So ist es plausibel, dass eine Erhöhung der Konzentration von Ribonukleasen im Plasma zur hydrolytischen Spaltung der Nukleinsäure führt und damit Ribonukleasen antikoagulatorische Wirkung entfalten. Da Endothelzellen vermehrt RNAse A produzieren und ins Blut sezernieren, wie an Zellkulturen gezeigt wurde (Landre et al., J. Cell. Biochem. 86, 540-552, 2002), kommt eine antikoagulatorische Therapie mit Ribonukleasen dem natürlichen Regulationsprinzip im Gefäßsystem sehr nahe. Alternativ könnten Ribonukleasen auch eine antikoagulatorische Wirkung gegenüber Ribozymen oder RNA-Aptameren entfalten, da diese Substanzen, ähnlich wie natürliche RNA, eine Kontaktphasenaktivierung bewirken könnten.

### Beispiel 6. Zelluläre Funktionen von RNA

Da RNA eine gewisse Überlebenszeit auch im Plasmamilieu hat, wurde die Wirkung dieses polyanionischen Materials auf Gefäßwandzellen, nämlich Endothelzell-Monolayer in Kultur, getestet. Ein sehr sensibler funktioneller Test für die zelluläre Wirkung von extrazellulärer RNA ist die Änderung der Endothelzell-Permeabilität, die an dichten Monolayern von mikrovaskulären zerebralen Schweineendothelien untersucht wurde. In konzentrations-abhängiger Weise erhöhte RNA die Permeabilität dieser Zellen, eine Vorinkubation mit RNAse konnte diese Wirkung von extrazellulärer RNA verhindern. Da eine Beeinflussung der Endothelzell-Permeabilität im Bereich einer Gefäßverletzungsstelle notwendig für die einsetzenden Wundheilungsreaktionen ist, wird dieser zellulären Funktion extrazellulärer RNA eine große Bedeutung zugemessen.

## Patentansprüche

1. Pharmazeutische Zubereitung, **dadurch gekennzeichnet, dass** sie eine zur Förderung der Blutgerinnung ausreichende Menge von natürlicher oder synthetischer RNA oder von einem oder mehreren die Blutgerinnung fördernden Bruchstücken natürlicher oder synthetischer RNA, Peptidnukleinsäuren, Ribozymen oder RNA-Aptameren, und einen Aktivator für einen plasmatischen Blutgerinnungsfaktor enthält.

2. Pharmazeutische Zubereitung nach Anspruch 1, **dadurch gekennzeichnet, dass** sie als Aktivator die den Faktor VII aktivierende Protease (=FSAP) oder ihr Proenzym enthält.

3. Pharmazeutische Zubereitung nach Anspruch 1, **dadurch gekennzeichnet, dass** sie als Aktivator Komponenten der Kontaktphase, insbesondere Faktor XII, Kininogen und/oder Präkallikrein, enthält.

4. Verwendung einer pharmazeutischen Zubereitung zur Herstellung eines Medikamentes zur Förderung der Blutgerinnung, **dadurch gekennzeichnet, dass** sie eine zur Förderung der Blutgerinnung ausreichende Menge von natürlicher oder synthetischer RNA oder von einem oder mehreren die Blutgerinnung fördernden Bruchstücken natürlicher oder synthetischer RNA, Peptidnukleinsäuren, Ribozyme oder RNA-Aptamere enthält.

5. Verwendung einer pharmazeutischen Zubereitung nach einem der Ansprüche 1 bis 3, zur Herstellung eines Medikamentes zur Förderung der Blutgerinnung.

6. Verwendung einer pharmazeutischen Zubereitung nach Anspruch 4 oder 5 für das Auslösen der extrinsischen Gerinnungskaskade.

7. Verwendung einer pharmazeutischen Zubereitung nach Anspruch 4 oder 5 für das Auslösen der intrinsischen Gerinnungskaskade.

8. Verwendung einher pharmazeutischen Zubereitung zur Herstellung eines Medikaments zur Hemmung der Koagulation und/oder Aufhebung oder Verhinderung der prokoagulatorischen Wirkung von RNA, **dadurch gekennzeichnet, dass** sie eine zur Hemmung der Koagulation ausreichende Menge einer oder mehrerer Ribonukleasen enthält, die die prokoagulatorische Wirkung von RNA aufhebt oder verhindert.

## Claims

1. Pharmaceutical preparation, **characterized in that** it comprises an amount, sufficient for promoting coagulation, of natural or synthetic RNA or of one or more coagulation-promoting fragments of natural or synthetic RNA, peptide-nucleic acids, ribozymes or RNA aptamers, and an activator for a plasma coagulation factor.

2. Pharmaceutical preparation according to Claim 1, **characterized in that** it comprises factor VII activating protease (= FSAP) or its proenzyme as activator.

3. Pharmaceutical preparation according to Claim 1, **characterized in that** it comprises components of the contact phase, particularly factor XII, kininogen and/or prekallikrein, as activator.

4. Use of a pharmaceutical preparation in the manufacture of a medicament for promoting coagulation, **characterized in that** it comprises an amount, sufficient for promoting coagulation, of natural or synthetic RNA or of one or more coagulation-promoting fragments of natural or synthetic RNA, peptide-nucleic acids, ribozymes or RNA aptamers.

5. Use of a pharmaceutical preparation according to any one of Claims 1 to 3 in the manufacture of a medicament for promoting coagulation.

6. Use of a pharmaceutical preparation according to Claim 4 or 5 for inducing the extrinsic coagulation cascade.

7. Use of a pharmaceutical preparation according to Claim 4 or 5 for inducing the intrinsic coagulation cascade.

8. Use of a pharmaceutical preparation in the manufacture of a medicament for inhibiting coagulation and/or abolishing or preventing the procoagulant effect of RNA, **characterized in that** it comprises an amount, sufficient to inhibit coagulation, of one or more ribonucleases which abolishes or prevents the procoagulant effect of RNA.

## Revendications

1. Préparation pharmaceutique, **caractérisée en ce qu'**elle contient une quantité, suffisante pour favoriser la coagulation du sang, d'ARN naturel ou synthétique ou d'un ou plusieurs fragments d'ARN naturel ou synthétique, peptides-acides nucléiques, ribozymes ou aptamères d'ARN, favorisant la coagulation du sang, et un activateur pour un facteur plasmatique de coagulation du sang.

2. Préparation pharmaceutique selon la revendication 1, **caractérisée en ce qu'**elle contient comme activateur la protéase activant le facteur VII (= FSAP) ou sa proenzyme.

3. Préparation pharmaceutique selon la revendication 1, **caractérisée en ce qu'**elle contient comme activateur des composants de la phase de contact, en particulier le facteur XII, le kininogène et/ou la prékallikréine.

4. Utilisation d'une préparation pharmaceutique pour la fabrication d'un médicament destiné à favoriser la coagulation du sang, **caractérisée en qu'**elle contient une quantité, suffisante pour favoriser la coagulation du sang, d'ARN naturel ou synthétique ou d'un ou plusieurs fragments d'ARN naturel ou synthétique, peptides-acides nucléiques, ribozymes ou aptamères d'ARN, favorisant la coagulation du sang.

5. Utilisation d'une préparation pharmaceutique selon l'une quelconque des revendications 1 à 3, pour la fabrication d'un médicament destiné à favoriser la coagulation du sang.

6. Utilisation d'une préparation pharmaceutique selon la revendication 4 ou 5, pour le déclenchement de la cascade de coagulation extrinsèque.

7. Utilisation d'une préparation pharmaceutique selon la revendication 4 ou 5, pour le déclenchement de la cascade de coagulation intrinsèque.

8. Utilisation d'une préparation pharmaceutique pour la fabrication d'un médicament destiné à inhiber la coagulation et/ou à supprimer ou inhiber l'action procoagulante d'ARN, **caractérisée qu'**elle contient une quantité, suffisante pour inhiber la coagulation, d'une ou plusieurs ribonucléases qui suppriment ou empêchent l'action procoagulante d'ARN.
